# DEMANDE DE BREVET EUROPEEN

(11) **EP 0 707 838 A1**
(43) Date de publication de la demande: **24.04.1996**
(21) Numéro de dépôt: 95420274.3
(22) Date de dépôt: 04.10.1995
(51) Int. Cl.: A61F 2/34, A61L 27/00

(54) **Implant cotyloidien pour prothèse de hanche**

(30) Priorité: 11.10.1994 FR 9412322
(71) Demandeur: MERCK BIOMATERIAL FRANCE, F-01800 Charnoz (FR); Bascoulergue, Gérard, F-62520 Le Touquet (FR)
(72) Inventeur: Bascoulergue, Gérard, F-62520 Le Touquet (FR); De Witte, Gérard, F-26300 Chateauneuf sur Isere (FR)
(74) Mandataire: Laurent, Michel

(57) **Abrégé**

Cet implant cotyloïdien pour prothèse de hanche, destiné à coopérer avec la tête de la tige fémorale de ladite prothèse est constitué d'une bande de frottement (6) réalisée en un matériau à dureté élevée, en forme sensiblement d'anneau ou de section d'anneau.

## Description

L'invention concerne un nouvel implant cotyloïdien, destiné à être mis en place au sein de la cavité acétabulaire ou cotyloïdienne de l'aile illiaque d'une articulation de la hanche, lors de la mise en place d'une prothèse totale de hanche.

Une prothèse totale de hanche est constituée de deux parties, respectivement une tige, destinée à être insérée dans le fémur à remplacer ou à renforcer, portant à son extrémité libre une tête généralement sphérique, et venant s'articuler dans la seconde partie constitutive de la prothèse, à savoir un cotyle, lui-même engagé et solidarisé à la cavité cotyloïdienne de l'aile illiaque considérée.

Un tel cotyle est généralement de forme sphérique ou hémisphérique, et peut être soit monobloc (le plus souvent en polyéthylène haute densité) et se fixe alors par l'intermédiaire d'un ciment acrylique, soit il comporte respectivement une cupule de soutien, destinée à être insérée et fixée dans la cavité cotyloïdienne proprement dite, et un noyau ou insert dit "de frottement", destiné à s'insérer le plus étroitement possible dans la cupule de maintien, de coapter le mieux possible à celle-ci, et dont la face interne de forme hémisphérique est destinée à recevoir la tête sphérique de la tige de la prothèse.

Un des problèmes majeurs auxquels sont confrontés les praticiens concerne les couples de friction inhérents aux frottements qui s'exercent entre la tête et le noyau tant en fonctionnement dynamique que statique de l'articulation.

On a par exemple proposé des surfaces de friction métal (tête) - polyéthylène (noyau). Le diamètre des têtes des tiges est plus ou moins important, mais ne peut toutefois dépasser certaines limites, compte tenu de l'épaisseur du polyéthylène constitutif du noyau, qui ne peut être inférieure à 7 mm, ce qui peut s'avérer gênant, dès lors que l'on souhaite utiliser des diamètres de tête plus importants, notamment en vue d'améliorer la stabilité de l'articulation prothésée.

On a également proposé des surfaces de friction métal-métal, mais qui ont engendré des problèmes d'adéquation entre la tête et le noyau, induisant eux-mêmes des descellements inhérents à l'augmentation du couple de friction. Afin de limiter ce phénonème, on a alors proposé des cotyles métalliques munis de bandes de glissement rapportées, ces bandes étant recouvertes d'un matériau à très faible coefficient de frottement, typiquement en polytétrafluoroéthylène (TEFLON : marque déposée).

Cependant, ces cotyles métalliques à bandes ont rapidement posés des problèmes dus à la libération de particules de TEFLON engendrant des intolérances, des descellements, etc.., conduisant à la suspension de leur utilisation.

On a également envisagé des couples de friction céramique-céramique. Cependant, les mêmes problèmes que pour les surfaces métal-métal sont apparus, inhérents au grippage conduisant à des descellements. Des solutions techniques ont permis d'améliorer ces paramètres, notamment par usinage des deux pièces constituant l'articulation prothétique (tête - cotyle), qui étaient obligatoirement appairées, puisque rodées l'une avec l'autre. Ces techniques ne sont toujours pas satisfaisantes, puisque outre les risques de mélange susceptible d'intervenir entre couples de pièces appairées, se greffe l'impossibilité de reprise de l'un seulement des deux composants constitutifs desdits couples.

Aucune solution n'est à ce jour satisfaisante. De fait, les couples de friction les plus utilisés sont généralement des couples céramique (tête) - polyéthylène (noyau). Cependant, le polyéthylène est un matériau qui subit des déformations plastiques et élastiques et en outre, il est sensible à l'usure engendrée par son couple de friction avec la tête en céramique, entraînant la formation de débris d'usure, et générant de manière connue une intolérance, l'augmentation du couple de friction, et son corollaire, des problèmes de descellements.

En d'autres termes, il n'existe pas à ce jour de couple de friction idéal pour les prothèses totales de hanche.

L'objet de l'invention est de s'affranchir de ces différents inconvénients en proposant une prothèse de hanche et plus spécifiquement un implant cotyloïdien, susceptible de diminuer les surfaces de friction et partant, les couples de friction correspondants, et de diminuer les inconvénients majeurs qu'ils engendrent.

Cet implant cotyloïdien pour prothèse de hanche, destiné à coopérer avec la tête de la tige fémorale de ladite prothèse se caractérise en ce qu'il est constitué d'une bande de frottement réalisée en un matériau dur, en forme sensiblement d'anneau ou de section d'anneau.

Par anneau, on entend au sens géométrique la surface séparant deux circonférences concentriques ou non, et notamment un anneau sphérique, voire un segment de surface.

De la sorte, la coaptation tête-implant n'est plus congruente : il n'y a plus frottement demi-sphère contre sphère, mais seulement un frottement limité au seul anneau sphérique.

On diminue de la sorte la surface de friction à environ 60 % de la surface normale de coaptation d'une sphère (la tête) au sein d'une demi-sphère (le noyau), et partant d'une quantité équivalente les couples de friction, limitant de manière drastique les risques d'arrachement et de descellement. Parallèlement, l'usure engendrée par la friction tête - anneau s'en trouve également limitée.

Avantageusement, le matériau constitutif de l'implant et de la tête est le même et est choisi dans le groupe comprenant le pyrocarbonne, les céramiques d'alumine ou de zircone, le cobalt chrome, etc. .

Selon une première forme de réalisation de l'invention, l'implant est directement fixé à la cavité cotyloïdienne par un ou plusieurs organes de fixation, par exemple constitués de plots d'ancrage, de vis etc...

Dans une autre version de l'invention, l'implant est solidarisé en superstructure à la cupule de frottement ou noyau d'un cotyle. En d'autres termes, il forme saillie par rapport à la surface hémisphérique interne de ladite cupule de frottement ou de celle de la cavité cotyloïdienne.

La manière dont l'invention peut être réalisée et les avantages qui en découlent ressortiront mieux des exemples de réalisation qui suivent donnés à titre indicatif et non limitatif à l'appui des figures annexées.

La figure 1 est une représentation schématique en éclaté de l'implant cotyloïdien selon une première forme de réalisation de l'invention.

La figure 2 est une représentation schématique d'une vue du dessus de l'implant cotyloïdien de la figure 1, en place au sein de la cavité acétabulaire, dont la figure 3 est une vue partiellement en section.

La figure 4 est une représentation schématique en perspective de l'implant cotyloïdien selon une seconde forme de réalisation de l'invention.

La figure 5 est une représentation schématique d'une vue du dessus de l'implant cotyloïdien de la figure 4, en place au sein de la cavité acétabulaire, dont la figure 6 est une vue partiellement en section.

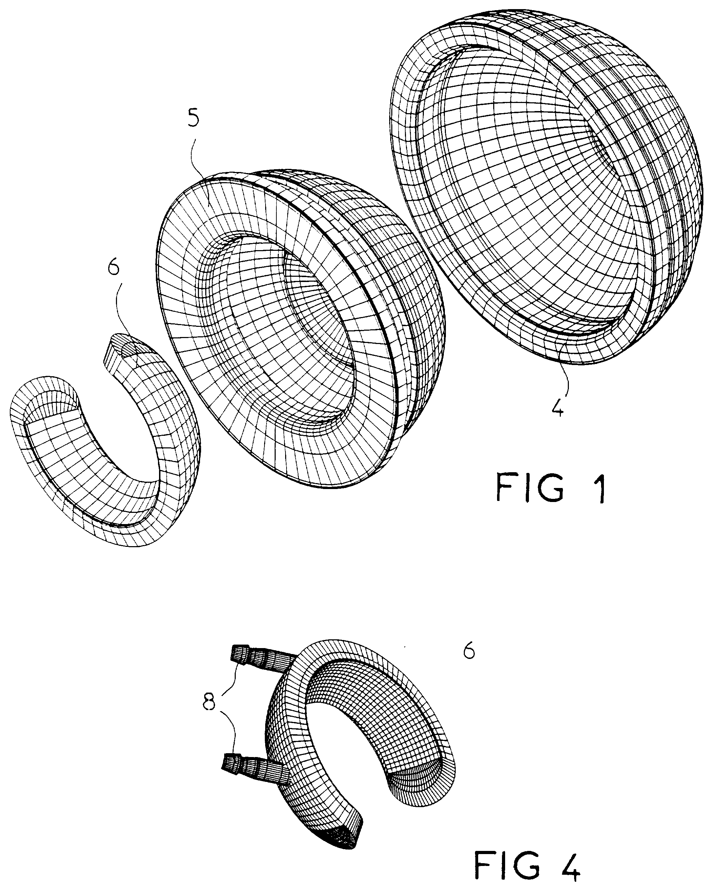

On a représenté sur les figures 1 à 3 une première forme de réalisation de l'invention, dans laquelle l'insert cotyloïdien est implanté dans la cupule de frottement (noyau) (5) d'un cotyle (4) traditionnel.

On a représenté sous la référence (1) l'aile illiaque de l'articulation considérée et référencé (2) le trou obturateur correspondant. De manière traditionnelle, le cotyle métallique (4), muni d'une cupule de frottement (5) réalisée en polyéthylène, est implanté au sein de la cavité cotyloïdienne ou acétabulaire (3) au moyen d'organes de fixation (7), typiquement constitués par des vis d'ancrage.

Selon une caractéristique fondamentale de l'invention, cette cupule polyéthylène (5) reçoit un implant cotyloïdien de friction (6) en forme sensiblement de U ou de section d'anneau sphérique, formant donc une protubérance ou une saillie par rapport à la surface interne hémisphérique de la cupule ou noyau de frottement (5). Cet implant est fixé par tout moyen au noyau (5), notamment par collage, voire par inclusion lors du thermomoulage du noyau.

De fait, cet implant de friction (6) et lui seul est destiné à coopérer avec la tête sphérique d'une tige de prothèse de hanche (non représentée) au niveau de la cavité cotyloïdienne de ladite articulation.

En fait, cet implant en forme de bande de frottement, limite la surface de friction tête - noyau aux seules zones réellement sollicitées lors du fonctionnement tant dynamique que statique de l'articulation considérée.

On aboutit donc à une surface prothétique non congruente, dans la mesure où seule une partie de la tête prothétique coopère par frottement avec la cavité cotyloïdienne.

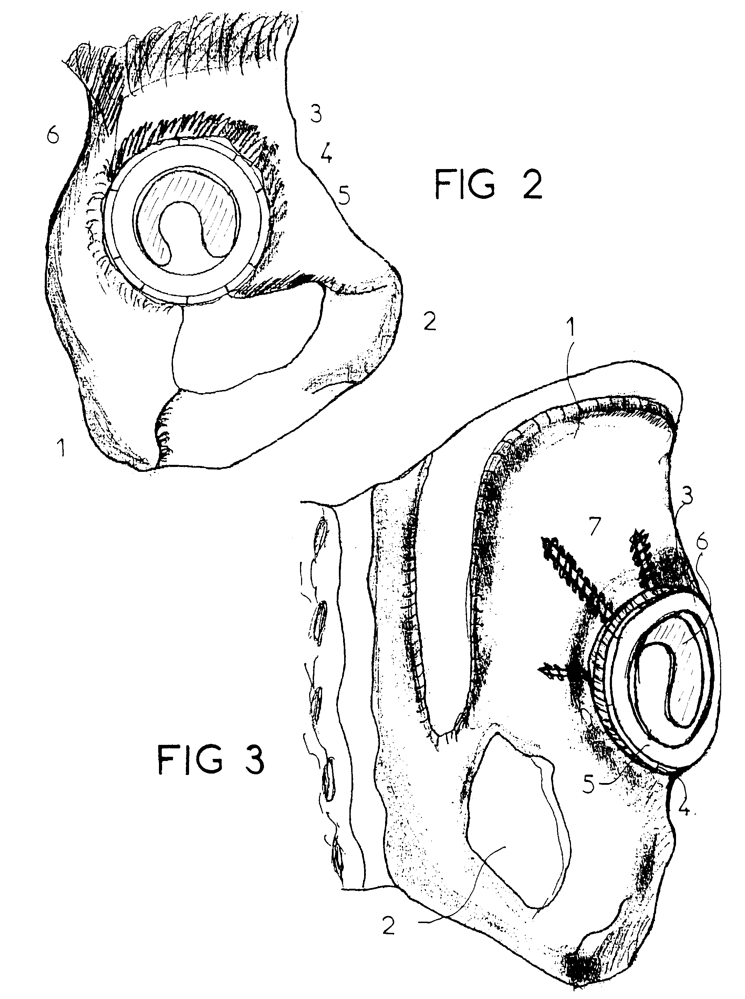

L'épaisseur de cette anneau ou bande de frottement acétabulaire (6) est par exemple de 2 mm par rapport à la surface interne du noyau de frottement (5). Le U est avantageusement positionné en zone superopostérieure, telle que représenté sur la figure 2, correspondant à la surface utile de frottement de la tête avec la cavité acétabulaire. Cependant, il peut être orientable, grace notamment aux multiples possibilités de positionnement du noyau auquel il est solidarisé, au sein de la cupule métallique.

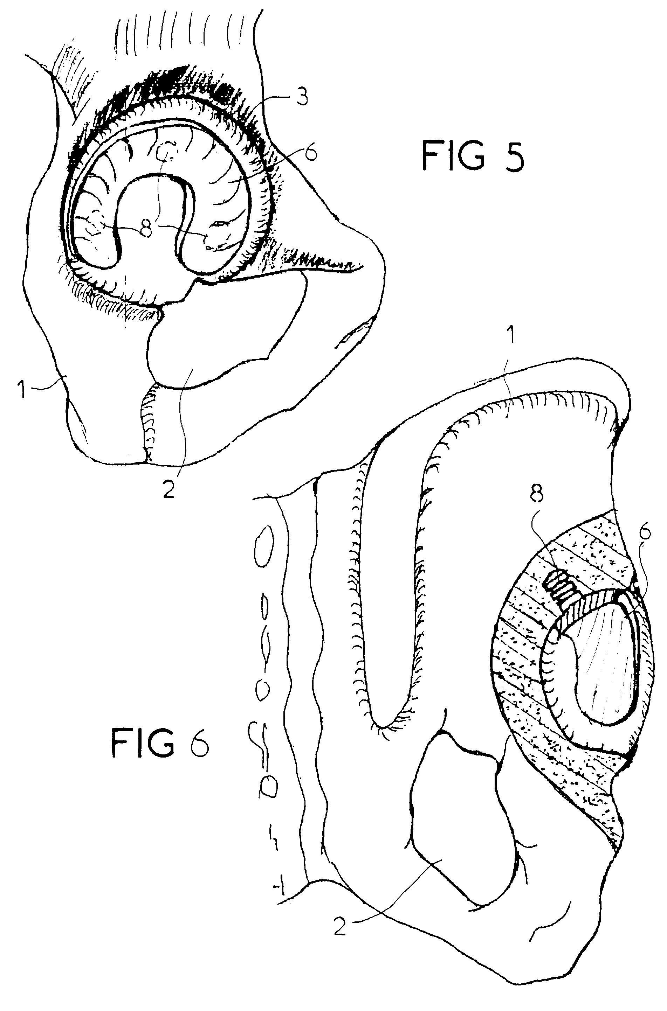

Selon une autre forme de réalisation de l'invention représentée en liaison avec les figures 4 à 6, l'implant de friction (6), également en forme de U ou de section d'anneau spéhrique, est directement fixé dans l'os acétabulaire et ce, par l'intermédiaire de moyens d'ancrage en eux-mêmes connus (8), et par exemple constitués par des plots d'ancrage. Dans ce cas, comme dans le cas précédent, seul l'implant (6) est en contact avec la tête sphérique de la tige de la prothèse.

Selon une caractérisitique avantageuse de l'invention, la tête prothétique et l'implant cotyloïdien sont réalisés en un même matériau dur, et par exemple en pyrocarbone, en céramique de zircone ou d'alumine, voire en cobalt chrome. La dureté du matériau mis en oeuvre est d'au moins 275, voire 350 HV (Essai Vickers).

De part la mise en place d'un tel implant, on élimine toutes les frictions parasites, toutes les obligations d'appairage, et on diminue drastiquement les phénomènes de grippage, d'usure, ces résultats étant en outre partiellement inhérents à la dureté des matériaux utilisés.

Compte-tenu de la diminution du couple de friction inhérent à la réduction des surfaces de friction, on diminue sinon élimine les risques d'arrachement et de descellement, notamment du cotyle ou de la tige prothétique.

Parallèlement, il est possible alors d'utiliser des têtes prothétiques de plus gros diamètre, concourant également à limiter voire annuler les risques de luxation.

## Revendications

1. Implant cotyloïdien pour prothèse de hanche, destiné à coopérer avec la tête de la tige fémorale de ladite prothèse *caractérisé* en ce qu'il est constitué d'une bande de frottement (6) réalisée en un matériau à dureté élevée, en forme sensiblement d'anneau ou de section d'anneau.

2. Implant cotyloïdien pour prothèse de hanche selon la revendication 1, *caractérisé* en ce qu'il est en forme de U.

3. Implant cotyloïdien pour prothèse de hanche selon l'une des revendications 1 et 2, *caractérisé* en ce que le matériau constitutif de l'implant est choisi dans le groupe comprenant le pyrocarbone, les céramiques d'alumine ou de zircone, le cobalt chrome.

4. Implant cotyloïdien pour prothèse de hanche selon l'une des revendications 1 à 3, *caractérisé* en ce que le matériau constitutif de l'implant est d'une dureté d'au moins 275 HV mesurée selon la technique Vickers.

5. Implant cotyloïdien pour prothèse de hanche selon l'une des revendications 3 et 4, *caractérisé* en ce que le matériau constitutif de la tête de la tige prothétique est le même que celui constitutif de l'implant (6).

6. Implant cotyloïdien pour prothèse de hanche selon l'une des revendications 1 à 5, *caractérisé* en ce qu'il est directement fixé à la cavité cotyloïdienne (3) par un ou plusieurs organes de fixation (8).

7. Implant cotyloïdien pour prothèse de hanche selon l'une des revendications 1 à 5, *caractérisé* en ce qu'il est solidarisé en superstructure au noyau (5) d'un cotyle (4).

8. Implant cotyloïdien pour prothèse de hanche selon l'une des revendications 1 à 7, *caractérisé* en ce qu'il est implanté en position supéro-postérieure au sein de la cavité cotyloïdienne ou au sein d'un cotyle.

9. Implant cotyloïdien pour prothèse de hanche selon l'une des revendications 1 à 8, *caractérisé* en ce qu'il forme saillie par rapport à la surface interne de la cavité cotyloïdienne ou par rapport à la surface interne du noyau de frottement (5) d'un cotyle (4).
